# EUROPEAN PATENT SPECIFICATION

(11) **EP 4 338 766 B1**
(45) Date of publication and mention of the grant of the patent: **11.03.2026**
(21) Application number: 22807063.7
(22) Date of filing: 18.02.2022
(51) Int. Cl.: A61L 29/14, A61M 25/10

(54) **BALLOON FOR BALLOON CATHETER AND METHOD FOR MANUFACTURING BALLOON CATHETER**
BALLON FÜR BALLONKATHETER UND VERFAHREN ZUR HERSTELLUNG EINES BALLONKATHETERS
BALLONNET POUR CATHÉTER À BALLONNET ET PROCÉDÉ POUR FABRICATION DE CATHÉTER À BALLONNET

(30) Priority: 10.05.2021 JP 2021079672
(43) Date of publication of application: 20.03.2024
(73) Proprietor: KANEKA CORPORATION, Osaka 530-8288 (JP)
(72) Inventor: NAKAMURA, Kokoro, Settsu-shi, Osaka 566-0072 (JP)
(74) Representative: Plasseraud IP
(86) International application number: PCT/JP2022/006686
(87) International publication number: WO 2022/239356

(56) References cited:
- EP-A1- 2 974 766
- WO-A1-2013/118807
- WO-A1-2014/141382
- JP-A- 2004 298 354
- JP-A- 2004 298 354
- JP-A- 2005 305 187
- JP-A- 2008 000 553
- JP-A- 2008 000 553
- JP-A- H0 938 195
- JP-B2- 3 766 122

## Description

### TECHNICAL FIELD

The present invention relates to a balloon for a balloon catheter and a method of manufacturing a balloon catheter.

### BACKGROUND ART

Angioplasty, in which a balloon catheter is inserted into a narrowed blood vessel to inflate the balloon to secure blood flow by dilating the blood vessel, is widely used as a minimally invasive therapy. The angioplasty is used, for example, to treat diseases such as myocardial infarction caused by stenosis in the coronary arteries of the heart, or to treat stenosis that occurs in the shunt for dialysis. A balloon of the balloon catheter is usually cylindrical in shape, with the distal and proximal sides tapered, and has a cylindrical straight tubular part, a proximal tapered part located proximal to the straight tubular part, and a distal tapered part located distal to the straight tubular part.

Usually, when a balloon catheter is used to dilate a stenotic area, the balloon is inflated at a pressure appropriate to the target site. However, if the balloon is over-pressurized during the procedure due to unexpected internal pressure being applied to the balloon etc., the balloon may break. If the balloon breaks in the circumferential direction, there is a serious risk that a fragment of the balloon distal to the breakage point will remain inside the body. Therefore, even if the balloon were to break, the technique is needed such that the balloon does not break in the circumferential direction, but break in the longitudinal axis direction.

For example, Patent documents 1 to 3 have proposed balloons that address the issue of making the balloon pressure-resistant and reducing circumferential fracture. The Patent document 1 discloses a balloon in which the difference in the ratio of circumferentially oriented polymer chains in the inflatable part is less than a predetermined value, the Patent document 2 discloses a balloon in which the ratio of the number of oriented distributions calculated by dividing the number of circumferentially oriented distributions by the number of axially oriented distributions in the tubular part is less than a predetermined value, and the patent document 3 discloses a balloon that breaks in the axial direction and is resistant to scattering rupture because the material of the balloon itself has molecular orientations that are aligned axially.

### RELATED ART DOCUMENT

### PATENT DOCUMENT

Patent Document 1: JP 2004-298354 A
Patent Document 2: WO 2014/141382
Patent Document 3: JP 2008-000553 A

### SUMMARY OF THE INVENTION

### PROBLEMS TO BE SOLVED BY THE INVENTION

However, although the above-described balloons tried to suppress circumferential fracture and improve pressure resistance by controlling the molecular orientation of the balloon, they could not suppress circumferential fracture by making longitudinal axial fracture while controlling the site where the longitudinal axial fracture occurs when the balloon breaks. Furthermore, when longitudinal axial fracture occurs, the longitudinal axial fracture may extend to the proximal or distal end side, which is relatively thick-walled, of the balloon to form a L-shaped crack, which is circumferential crack at the proximal or distal end sides, causing a risk that a broken piece remains in the body. However, conventional balloons are not sufficient to prevent such a L-shaped crack.

In view of the above circumstances, the objective of the present invention is to provide a balloon for a balloon catheter that can suppress circumferential fracture by allowing the balloon to generate fracture in the longitudinal axis direction in the central part of the balloon even if the balloon breaks. Another objective of the present invention is to provide a balloon for a balloon catheter that can prevent longitudinal axial fracture from extending to the proximal or distal end side of the balloon to form a L-shaped crack that is circumferential fracture at the end part by keeping the longitudinal axial fracture within the straight tubular part. These objectives are achieved according to the invention by means of a balloon for a balloon catheter according to claim 1, a catheter comprising such a balloon according to claim 6, and a method of manufacturing such a catheter according to claim 7. Preferred embodiments are defined in the dependent claims.

### MEANS FOR SOLVING THE PROBLEMS

According to the invention, one embodiment of the balloon for a balloon catheter that solves the above problems is a balloon that is made of a resin having molecular orientation and has a longitudinal axis direction and a circumferential direction that is along a circumference of the balloon in an inflated state in a cross section perpendicular to the longitudinal axis direction, comprising: a straight tubular part, a proximal tapered part located proximal to the straight tubular part, and a distal tapered part located distal to the straight tubular part, wherein a proximal end of the straight tubular part is defined as a position of 0%, and distal end of the straight tubular part is defined as a position of 100% in the longitudinal axis direction; a main orientation direction of the molecular orientation in a proximal section from the position of 0% to a position of 10% and a distal section from a position of 90% to the position of 100% is the circumferential direction; and a longitudinal axis direction component of the molecular orientation in a central section from a position of 40% to a position of 60% is more than a longitudinal axis direction component of the molecular orientation in the proximal section and the distal section. Thus, the longitudinal axis direction component of the molecular orientation in the central section is more than the longitudinal axis direction component of the molecular orientation in the proximal section and the distal section, which allows the central section to trigger longitudinal axial fracture even if the balloon breaks due to overpressurization, etc., whereby allowing internal pressure to be released by generating the longitudinal axial fracture in the central section to prevent circumferential fracture. In addition, if longitudinal axial fracture generated in the central section runs vigorously in the longitudinal axis direction beyond the proximal and distal sections, a L-shaped crack, which becomes circumferential fracture in the relatively thick-walled proximal and distal tapered parts. However, the balloon for a balloon catheter according to the present invention can prevent the longitudinal axial fracture generated in the central section from running vigorously beyond the proximal and distal sections to the proximal and distal tapered parts even if the longitudinal axial fracture reaches the proximal and distal sections, because the main orientation direction of the molecular orientation in the proximal and distal sections is the circumferential direction. Thus, the longitudinal axial fracture can be kept within the straight tubular part, preventing a L-shaped crack that is circumferential fracture in the proximal and distal tapered parts. As a result, the risk that a broken piece remains in the body caused by circumferential fracture or L-shaped crack can be avoided.

Preferably, in the above balloon for a balloon catheter, a main orientation direction of the molecular orientation in the central section is the longitudinal axis direction.

Preferably, in the above balloon for a balloon catheter, the longitudinal axis direction component of the molecular orientation gradually decreases from the central section toward the position of 0% and gradually decreases from the central section toward the position of 100%.

Preferably, in the above balloon for a balloon catheter, a main orientation direction of the molecular orientation in the central section is the longitudinal axis direction, and when a section from the position of 10% to a position of 40% is defined as a proximal central section and a section from a position of 60% to the position of 90% is defined as a distal central section, a main orientation direction of the molecular orientation in the proximal central section and the distal central section changes from the longitudinal axis direction to the circumferential direction.

Preferably, in the above balloon for a balloon catheter, a film thickness of the balloon in the central section is thinner than a film thickness of the balloon in the proximal section and the distal section.

The present invention also provides a balloon catheter having the above balloon for a balloon catheter.

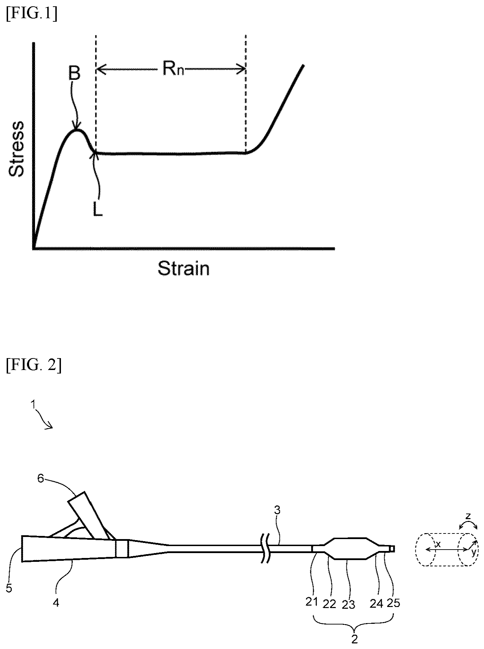

The present invention further provides a method of manufacturing the above balloon catheter. One embodiment of the method of manufacturing the balloon catheter that can solve the above problems has a step of preparing a parison made of a resin; a step of preparing a mold having a lumen and inner wall surface forming the lumen, the inner wall surface having a straight tubular part, a proximal tapered part located proximal to the straight tubular part, and a distal tapered part located distal to the straight tubular part; a step of placing the parison in the mold; a step of a first extending process in which the parison is extended in the longitudinal axis direction until exceeding a necking region of a stress-strain curve while the mold is heated; and a step of a second extending process in which, after the first extending process, the parison, which has been extended until exceeding the necking region, is further extended in the longitudinal axis direction with a higher internal pressure of the parison than in the first extending process while the mold is heated. For many resins, in the stress-strain curve as shown in FIG. 1, stress acts to elongate the molecular chains that have been bent in the region of elastic deformation up to a yield point B. After the yield point B, plastic deformation begins, in which the molecular chains having been attracted to each other by intermolecular forces shift in the shear direction. Once the molecular chains start to shift, some resins show a phenomenon where the molecular chains become loose and the stress decreases to a lower yield point L. After that, a region of flat stress is observed for a while, and this region is usually referred to as a necking region Rₙ. While a constant stress is seen as the molecular chains are displaced by strain in the necking region Rₙ, above a predetermined strain, the molecular chains are brought closer together and closely oriented, creating a strong intermolecular force between the molecular chains, so that the stress rises steadily in the region above the predetermined strain, that is, beyond the necking region Rₙ. By extending the parison in the longitudinal axis direction until exceeding the necking region Rₙ in the first extending process, and further extending the parison, which has been extended until exceeding the necking region Rₙ, in the longitudinal axis direction with a higher internal pressure in the following second extending process, it is possible to produce a balloon catheter with the balloon for a balloon catheter in which the main orientation direction of the molecular orientation in the proximal and distal sections is the circumferential direction and a longitudinal axis direction component of the molecular orientation in the central section is more than a longitudinal axis direction component of the molecular orientation in the proximal and distal sections.

Preferably, in the above manufacturing method, the parison is pressurized at a lower pressure in the first extending process than in the second extending process, and is further pressurized after exceeding the necking region in the second extending process.

The above manufacturing method preferably comprises a step of heating the mold so that a center portion of the straight tubular part of the mold is the hottest.

### EFFECTS OF THE INVENTION

According to the above balloon for a balloon catheter and the method of manufacturing the balloon catheter, circumferential fracture can be prevented by allowing the balloon to generate fracture in the longitudinal axis direction in the central part of the balloon even if the balloon breaks. In addition, the longitudinal axial fracture can be prevented from extending to the proximal or distal sides of the balloon to form a L-shaped crack that is circumferential fracture at the end part by keeping the longitudinal axial fracture within the straight tubular part. Therefore, even if the balloon breaks due to overpressurization, etc., the risk that a broken piece remains in the body caused by circumferential fracture or L-shaped crack can be avoided.

### BRIEF DESCRIPTION OF THE DRAWINGS

FIG. 1 is a stress-strain curve of polyester resin.
FIG. 2 is a side view of a balloon catheter in accordance with one embodiment of the present invention.
FIG. 3 is a plan view of a balloon in accordance with one embodiment of the present invention with a longitudinal fracture.
FIG. 4 is a plan view showing an example of a circumferential fracture in a balloon.
FIG. 5 is a plan view showing an example of a L-shaped crack in a balloon.
FIG. 6 is a plan view showing another example of a L-shaped crack in a balloon.
FIG. 7 is a diagram showing a method of preparing a sample for measuring the molecular orientation of the straight tubular part of a balloon in accordance with one embodiment of the present invention.
FIG. 8 is a contour diagram obtained by measuring a balloon in accordance with one embodiment of the present invention with a two-dimensional birefringence evaluation system.
FIG. 9 is a phase difference graph obtained by measuring a balloon in accordance with one embodiment of the present invention with a two-dimensional birefringence evaluation system.
FIG. 10 is an axial direction graph obtained by measuring a balloon in accordance with one embodiment of the present invention with a two-dimensional birefringence evaluation system.
FIG. 11 is a cross-sectional view showing a condition where a parison is placed in a mold in accordance with one embodiment of the present invention.
FIG. 12 is a cross-sectional view showing a condition in the first extending process in accordance with one embodiment of the present invention.
FIG. 13 is a cross-sectional view showing a condition in the second extending process in accordance with one embodiment of the present invention.
FIG. 14 is a cross-sectional view showing a condition after finishing the second extending process in accordance with one embodiment of the present invention.
FIG. 15 is a contour diagram of a balloon obtained in Example 1.
FIG. 16 is a phase difference graph of a balloon obtained in Example 1.
FIG. 17 is an axial direction graph of a balloon obtained in Example 1.
FIG. 18 is a graph of film thickness of a balloon obtained in Example 1.
FIG. 19 is a contour diagram of a balloon obtained in Comparative Example 1.
FIG. 20 is a phase difference graph of a balloon obtained in Comparative Example 1.
FIG. 21 is an axial direction graph of a balloon obtained in Comparative Example 1.
FIG. 22 is a graph of film thickness of a balloon obtained in Comparative Example 1.

### MODE FOR CARRYING OUT THE INVENTION

Hereinafter, the defibrillation catheter and the defibrillation system according to the present invention will be described based on the following embodiments, however, the present invention is not limited by the following embodiments and can be altered in design within a scope in compliance with the intent described above and below, and all the changes are to be encompassed within a technical scope of the present invention. Note that, in each drawing, hatching, reference signs for components, and the like may be omitted for convenience of description, and in such a case, the specification and other drawings are to be referred to. Furthermore, since the dimensions of the various components in the drawings are provided for the purpose of facilitating the understanding of the feature of the present invention, the dimensions may differ from the actual dimensions in some cases.

### 1. Balloon for balloon catheter

A balloon for a balloon catheter in accordance with embodiments of the present invention is characterized in that the balloon is made of a resin having molecular orientation, has a longitudinal axis direction and a circumferential direction that is along a circumference of the balloon in an inflated state in a cross section perpendicular to the longitudinal axis direction, and has a straight tubular part, a proximal tapered part located proximal to the straight tubular part, and a distal tapered part located distal to the straight tubular part, wherein a proximal end of the straight tubular part is defined as a position of 0% and a distal end of the straight tubular part is defined as a position of 100% in the longitudinal axis direction, a main orientation direction of the molecular orientation in a proximal section from the position of 0% to a position of 10% and a distal section from a position of 90% to the position of 100% is the circumferential direction, and a longitudinal axis direction component of the molecular orientation in a central section from a position of 40% to a position of 60% is more than a longitudinal axis direction component of the molecular orientation in the proximal section and the distal section. Thus, the longitudinal axis direction component of the molecular orientation in the central section is more than the longitudinal axis direction component of the molecular orientation in the proximal section and the distal section, which allows the central section to trigger longitudinal axial fracture even if the balloon breaks due to overpressurization, etc., whereby allowing internal pressure to be released by generating the longitudinal axis fracture in the central section to prevent circumferential fracture. In addition, if longitudinal axial fracture generated in the central section runs vigorously in the longitudinal axis direction beyond the proximal and distal sections, a L-shaped crack, which becomes circumferential fracture in the relatively thick-walled proximal and distal tapered parts. However, the balloon for a balloon catheter in accordance with embodiments of the present invention can prevent the longitudinal axial fracture generated in the central section from running vigorously beyond the proximal and distal sections to the proximal and distal tapered parts even if the longitudinal axial fracture reaches the proximal and distal sections, because the main orientation direction of the molecular orientation in the proximal and distal sections is the circumferential direction. Thus, the longitudinal axial fracture can be kept within the straight tubular part, preventing a L-shaped crack that is circumferential fracture in the proximal and distal tapered parts. As a result, the risk that a broken piece remains in the body caused by circumferential fracture or a L-shaped crack can be avoided. In the present specification, the balloon for a balloon catheter is sometimes referred to as merely "balloon".

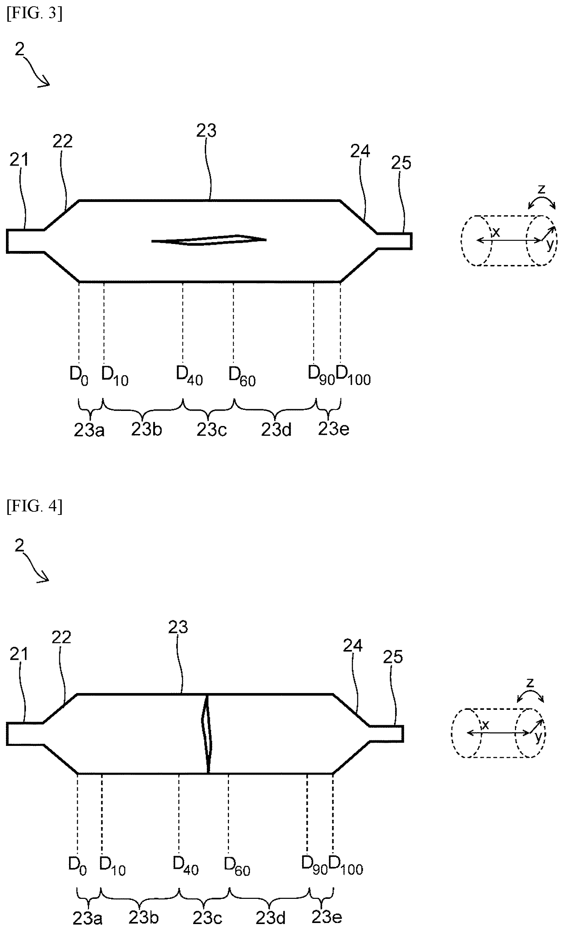

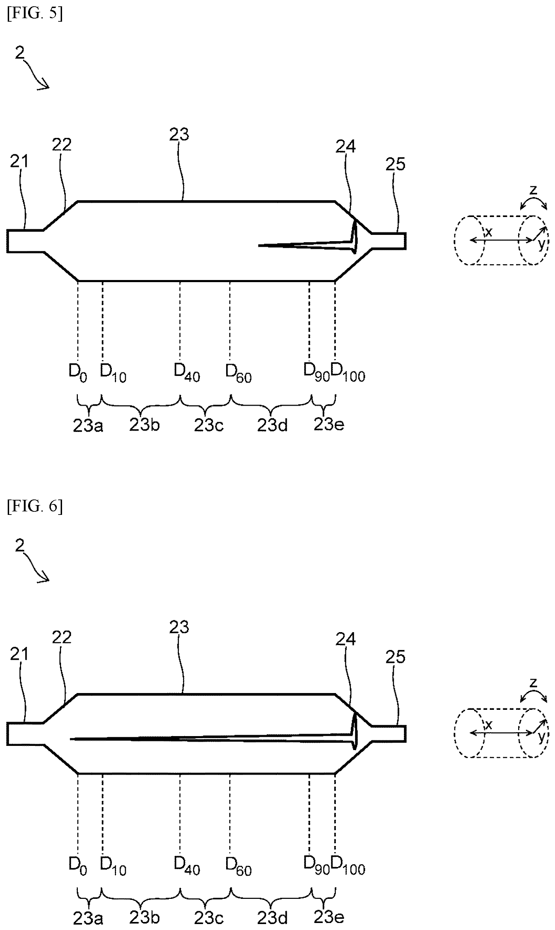

Referring to FIG. 2 to FIG. 6, the balloon for a balloon catheter will be described. FIG. 2 is a side view of a balloon catheter in accordance with one embodiment of the present invention. FIG. 3 is a plan view of a balloon in accordance with one embodiment of the present invention with a longitudinal fracture, FIG. 4 is a plan view showing an example of a circumferential fracture in a balloon, FIG. 5 is a plan view showing an example of a L-shaped crack in a balloon, and FIG. 6 is a plan view showing another example of a L-shaped crack in a balloon.

In the present invention, a proximal side refers to the direction towards a user's or operator's hand in the extending direction of a balloon catheter 1 or a longitudinal axis direction x of a shaft 3, and a distal side refers to the opposite side of the proximal side, that is, the direction towards the person to be treated. Members other than long-shaped members, such as shaft 3, also have the same longitudinal axis direction x as the shaft 3. The direction connecting the center of a balloon 2 and a point on the circumscribed circle of the balloon 2 in an inflated state in a cross section perpendicular to the longitudinal axis direction x is referred to as a radial direction y, and the direction along the circumference of the balloon 2 in an inflated state in a cross section perpendicular to the longitudinal axis direction x, i.e., in a cross section of the radial direction y is referred to as a circumferential direction z.

As shown in FIG. 2, the balloon catheter 1 has the shaft 3 and the balloon 2 disposed on a distal side of the shaft 3. The balloon 2 has the longitudinal axis direction x, the radial direction y, and the circumferential direction z, and is preferably formed into a tubular shape having openings on the proximal and distal sides. The balloon 2 is made of a resin having molecular orientation.

The balloon catheter 1 is configured such that fluid is introduced into the balloon 2 through the shaft 3, and the inflation and deflation of the balloon 2 can be controlled using an indeflator (compressor/ decompressor for balloons). The fluid may be a pressurized fluid pressurized by a pump or the like.

The balloon 2 has a straight tubular part 23, a proximal tapered part 22 located proximal to the straight tubular part 23, and a distal tapered part 24 located distal to the straight tubular part 23. The straight tubular part 23 preferably has approximately the same diameter along the longitudinal axis direction x, and the proximal tapered part 22 and the distal tapered part 24 are preferably formed so that the diameter decreases as it is away from the straight tubular part 23. When the balloon 2 is inflated at a lesion such as a stenosis, the straight tubular part 23 having the largest diameter can make sufficient contact with the stenosis to facilitate treatment such as dilation of the stenosis. The proximal tapered part 22 and the distal tapered part 24 having reduced diameters allow the outer diameter of the proximal and distal end parts of the balloon 2 to be reduced when the balloon is deflated to reduce the step between the shaft 3 and the balloon 2, making the balloon 2 easier to insert and pass within the body cavity.

The balloon 2 may have a proximal sleeve part 21 located proximal to the proximal tapered part 22 and may have a distal sleeve part 25 located distal to the distal tapered part 24. At least a part of the proximal sleeve part 21 and the distal sleeve part 25 can be fixed to the shaft 3.

When the proximal end of the straight tubular part 23 is defined as a position of 0% D₀ and the distal end of the straight tubular part 23 is defined as a position of 100% D₁₀₀ in the longitudinal axis direction x, a main orientation direction of the molecular orientation in a proximal section 23a from the position of 0% D₀ to a position of 10% D₁₀ and a distal section 23e from a position of 90% D₉₀ to the position of 100% D₁₀₀ is the circumferential direction z, and a component in the longitudinal axis direction x of the molecular orientation in a central section 23c from a position of 40% D₄₀ to a position of 60% D₆₀ is more than a component in the longitudinal axis direction x of the molecular orientation in the proximal section 23a and the distal section 23e.

The configuration in which the longitudinal axis direction component of the molecular orientation in the central section 23c is more than the longitudinal axis direction component of the molecular orientation in the proximal section 23a and the distal section 23e allows the central section 23c to trigger fracture in the longitudinal axis direction x even if the balloon 2 breaks due to overpressurization, etc. As shown in FIG. 3, this allows internal pressure to be released by generating fracture in the longitudinal axis direction x in the central section 23c, whereby preventing fracture in the circumferential direction z shown in FIG. 4 or preventing fracture in the longitudinal axis direction x generated at the end part from reaching the tapered part to form a L-shaped crack shown in FIG. 5.

In addition, if fracture in the longitudinal axis direction x generated in the central section 23c runs vigorously in the longitudinal axis direction x beyond the proximal section 23a and the distal section 23e, fracture in the circumferential direction z may be induced at the proximal tapered part 22 and the distal tapered part 24 to form a L-shaped crack as shown in FIG. 6. However, since the main orientation direction of the molecular orientation in the proximal section 23a and the distal section 23e is the circumferential direction z, even if fracture in the longitudinal axis direction x generated in the central section 23c reaches the proximal section 23a and the distal section 23e, the fracture in the longitudinal axis direction x can be prevented from reaching the proximal tapered part 22 and the distal tapered part 24 beyond the proximal section 23a and the distal section 23e. This can keep the fracture in the longitudinal axis direction x within the straight tubular part 23, preventing a L-shaped crack that is circumferential fracture in the relatively thick-walled proximal tapered part 22 and distal tapered part 24.

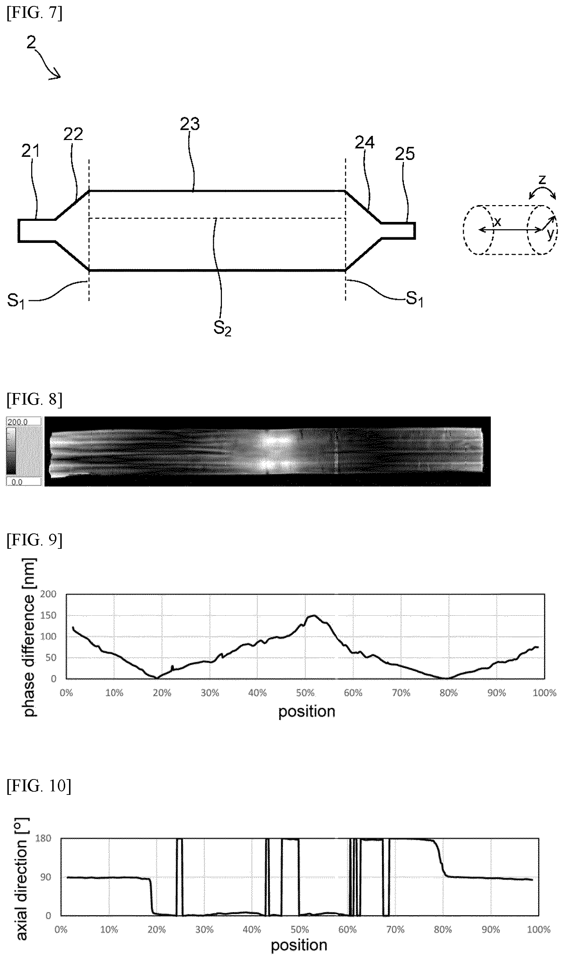

Referring to FIG. 7 to FIG. 10, a method of measuring the molecular orientation of the straight tubular part 23 will be described. FIG. 7 is a diagram showing a method of preparing a sample for measuring the molecular orientation of the straight tubular part 23 of the balloon 2 in accordance with one embodiment of the present invention. FIG. 8 is an example of a contour diagram obtained by measuring a balloon in accordance with one embodiment of the present invention with a two-dimensional birefringence evaluation system manufactured by Photonic Lattice, Inc., and FIG. 9 and FIG. 10 are examples of a phase difference graph and an axial direction graph, respectively, obtained as a result of line analysis in the longitudinal axis direction.

The molecular orientation of the straight tubular part 23 can be obtained by measuring a rectangular sample of the straight tubular part 23 using a two-dimensional birefringence evaluation system manufactured by Photonic Lattice, Inc. The rectangular sample can be obtained, as shown in FIG. 7, by cutting off the proximal tapered part 22 and the distal tapered part 24 from the balloon 2 along a first cut line S₁, and cutting the resulting straight tubular part 23 open along a second cut line S₂ in the longitudinal axis direction x.

An example of a contour diagram resulting from the measurement is shown in FIG. 8, and examples of a phase difference graph and an axial direction graph obtained as a result of line analysis in the longitudinal axis direction x are shown in FIG. 9 and FIG. 10, respectively. In each figure, the left end corresponds to the position of 0% D₀ of the straight tubular part 23 and the right end corresponds to the position of 100% D₁₀₀ of the straight tubular part 23. The contour diagram shows the magnitude of the phase difference in terms of color contrast, and the orientation state can be visually observed. Information on the strength of orientation can be specifically obtained from the phase difference graph, and information on the orientation direction can be specifically obtained from the axial direction graph.

The main orientation direction can be determined from the axial direction graph. In a given section, when the length of the line included in the range of 80° to 100° is longer than the length of the line included in the range of 0° to 10° and the range of 170° to 180°, the main orientation direction in the given section is the circumferential direction z. Conversely, in a given section, when the length of the line included in the range of 80° to 100° is shorter than the length of the line included in the range of 0° to 10° and the range of 170° to 180°, the main orientation direction in the given section is the longitudinal axis direction x. The intensity of the orientation component can be obtained from the phase difference graph. To determine which section of the proximal section 23a, the central section 23c, and the distal section 23e has more orientation direction component in the longitudinal axis direction x, the lengths of the lines included in the range of 0° to 10° and the range of 170° to 180° in the respective sections in the axial direction graph are compared, and the section with the longest line included in the range can be determined as the section with more orientation direction component in the longitudinal axis direction x. For example, in the balloon 2 showing the measurement results shown in FIG. 8 to FIG. 10, the axial direction graph indicates that the main orientation direction of the molecular orientation in the proximal section 23a and the distal section 23e is the circumferential direction z. It is also indicated that the main orientation direction of the molecular orientation in the central section 23c is the longitudinal axis direction x, and that the molecular orientation component in the longitudinal axis direction x in the central section 23c is more than the molecular orientation component in the longitudinal axis direction x in the proximal section 23a and the distal section 23e. Furthermore, the phase difference graph indicates that the orientation intensity decreases from the central section 23c to the proximal and distal sides, i.e., the molecular orientation component in the longitudinal axis direction x decreases.

The main orientation direction of the molecular orientation in the central section 23c is preferably the longitudinal axis direction x. The main orientation direction in the longitudinal axis direction x makes it easier to trigger fracture in the longitudinal axis direction x in the central section 23c even if the balloon 2 breaks due to overpressurization, etc. This allows internal pressure to be released by generating the fracture in the longitudinal axis direction x in the central section 23c, thus preventing fracture in the circumferential direction z more easily.

Preferably, the molecular orientation component in the longitudinal axis direction x gradually decreases from the central section 23c towards the position of 0% D₀ and gradually decreases from the central section 23c towards the position of 100% D₁₀₀. This allows the molecular orientation component in the longitudinal axis direction x to be greatest in the central section 23c, making it easier to trigger fracture in the longitudinal axis direction x in the central section 23c and to prevent fracture in the circumferential direction z because the fracture in the longitudinal axis direction x generated in the central section 23c allows internal pressure to be released. Gradual decrease of the molecular orientation component in the longitudinal axis direction x from the central section 23c towards the position of 0% D₀ means that, for example, the molecular orientation component in the longitudinal axis direction x of the position of 40% D₄₀, a position of 20%, and the position of 0% D₀ decreases in this order. Gradual decrease of the molecular orientation component in the longitudinal axis direction x from the central section 23c towards the position of 100% D₁₀₀ means that, for example, the molecular orientation component in the longitudinal axis direction x of the position of 60% D₆₀, a position of 80%, and the position of 100% D₁₀₀ decreases in this order. Thus, the molecular orientation component in the longitudinal axis direction x does not necessarily have to decrease continuously from the central section 23c to the proximal or distal side. Alternatively, the molecular orientation component in the longitudinal axis direction x may decrease continuously from the central section 23c to the proximal or distal side.

When a section from the position of 10% D₁₀ to the position of 40% D₄₀ is defined as a proximal central section 23b, and a section from the position of 60% D₆₀ to the position of 90% D₉₀ is defined as a distal central section 23d, the main orientation direction of the molecular orientation in the proximal central section 23b and the distal central section 23d preferably changes from the longitudinal axis direction x to the circumferential direction z. The change in the main orientation direction of the molecular orientation from the longitudinal axis direction x to the circumferential direction z in the proximal central section 23b and the distal central section 23d makes it easier to keep fracture within the straight tubular part 23 because the progression of the fracture in the longitudinal axis direction x generated in the central section 23c can be easily stopped in the proximal central section 23b and the distal central section 23d. The change in the main orientation direction of the molecular orientation can be learnt from the axial direction graph. The example shown in FIG. 10 indicates that the main orientation direction of the molecular orientation changes from the longitudinal axis direction x to the circumferential direction z in the proximal central section 23b and the distal central section 23d.

The film thickness of the balloon 2 in the central section 23c is preferably thinner than the film thickness of the balloon 2 in the proximal section 23a and the distal section 23e. The thinner film thickness in the central section 23c makes it easier to trigger fracture in the longitudinal axis direction x in the central section 23c, and thus, making it easy to keep the fracture in the longitudinal axis direction x within the central section 23c.

Although not shown in the figures, the balloon 2 may have a protrusion part that projects outwardly in the radial direction y from the outer surface and extends in the longitudinal axis direction x. The protrusion part is preferably provided on the outer surface of the balloon 2 in a pattern such as dots, lines, or net-shape. Disposing the protrusion part on the outer surface of the balloon 2 can provide a function of scoring to the protrusion part to incise and dilate a calcified stenosis in angioplasty. It also makes it possible to increase the strength of the balloon 2 and prevent overinflation when being pressurized.

Materials forming the balloon 2 include, for example, polyolefin-based resin such as polyethylene, polypropylene, ethylene-propylene copolymer; polyester-based resin such as polyethylene terephthalate and polyester elastomer; polyurethane-based resin such as polyurethane and polyurethane elastomer; polyphenylene sulfide-based resin; polyamide-based resin such as polyamide and polyamide elastomer; fluorine-based resin; silicone-based resin; and natural rubber such as latex rubber. Only one of these may be used, or two or more may be used in combination. Of these, polyamide-based resin, polyester-based resin, and polyurethane-based resin are preferably used. In particular, elastomer resin is preferably used from the viewpoint of thinning and flexibility of the balloon 2. For example, among polyamide-based resins, nylon 12, nylon 11, and the like are suitable for the resin forming the balloon 2, and more preferably nylon 12 because it is relatively easy to mole when blow molding. From the viewpoint of thinning and flexibility of the balloon 2, polyamide elastomers such as polyether ester amide elastomer and polyamide ether elastomer are preferably used. Of these, polyether ester amide elastomer is preferred in terms of high yield strength and good dimensional stability of the balloon 2.

The balloon 2 can be manufactured by placing a parison made of the above-described material in a mold, and biaxially stretching blow molding it. A preferred method of manufacturing the balloon 2 will be described below in the section "3. Method of manufacturing balloon catheter".

### 2. Balloon catheter

A balloon catheter of the present invention has the above-described balloon for a balloon catheter. A balloon catheter in accordance with embodiments of the present invention can be understood referring to the above section "1. Balloon for balloon catheter" and FIG. 2.

Materials forming the shaft 3 include, for example, polyamide-based resin, polyester-based resin, polyurethane-based resin, polyolefin-based resin, fluorine-based resin, polyvinyl chloride-based resin, silicone-based resin, and natural rubber. Only one of these may be used, or two or more may be used in combination. Of these, the material forming the shaft 3 is preferably at least one of polyamide-based resin, polyolefin-based resin, and fluorine-based resin. This can improve surface slipperiness of the shaft 3 and improve the insertion of a balloon catheter 1 into the body cavity.

The balloon 2 and the shaft 3 may be joined by adhesive bonding, welding, or by attaching a ring-shaped member at the point where the end of the balloon 2 and the shaft 3 overlap to swage them. Of these, the balloon 2 and the shaft 3 are preferably joined by welding. By welding the balloon 2 and the shaft 3, the bond between the balloon 2 and the shaft 3 is difficult to be released even when the balloon 2 is repeatedly inflated and deflated, easily increasing the strength of the bond between the balloon 2 and the shaft 3.

As shown in FIG. 2, the balloon catheter 1 may be provided with a hub 4 at a proximal side of the shaft 3, and the hub 4 may be provided with a fluid inlet 6 that is connected to the flow channel of the fluid supplied to the interior of the balloon 2. In addition, the hub 4 preferably has a guidewire insertion port 5 that is connected to the guidewire insertion channel. Such a configuration can facilitate the operation of supplying the fluid inside the balloon 2 to inflate and deflate the balloon 2 and delivering the balloon catheter 1 to a lesion site along a guidewire. While FIG. 2 shows the balloon catheter 1, which is a so-called over-the-wire type in which the guidewire is inserted over the distal to proximal side of the shaft 3, the balloon 2 in accordance with embodiments of the present invention can be also applicable to a so-called rapid exchange balloon catheter in which the guidewire is inserted from the distal side to the midway of the proximal side of the shaft 3.

The shaft 3 and the hub 4 may be joined by, for example, adhesive bonding or welding. Of these, the shaft 3 and the hub 4 are preferably joined by adhesive bonding. The adhesive bonding of the shaft 3 and the hub 4 can increase the bonding strength of the shaft 3 and the hub 4 to increase durability of the balloon catheter 1 when the materials forming the shaft 3 and the hub 4 are different, for example, in a case where the shaft 3 is made of material having high flexibility and the hub 4 is made of material having high stiffness.

### 3. Method of manufacturing balloon catheter

A method of manufacturing the balloon catheter in accordance with one embodiment of the present invention is characterized in that the method has a step of preparing a parison made of a resin; a step of preparing a mold having a lumen and inner wall surface forming the lumen, the inner wall surface having a straight tubular part, a proximal tapered part located proximal to the straight tubular part, and a distal tapered part located distal to the straight tubular part; a step of placing the parison in the mold; a step of a first extending process in which the parison is extended in the longitudinal axis direction until exceeding a necking region of a stress-strain curve while the mold is heated; and a step of a second extending process in which, after the first extending process, the parison, which has been extended until exceeding the necking region, is further extended in the longitudinal axis direction with a higher internal pressure of the parison than in the first extending process while the mold is heated. For many resins, in the stress-strain curve as shown in FIG. 1, stress acts to elongate the molecular chains that have been bent in the region of elastic deformation up to a yield point B. After the yield point B, plastic deformation begins, in which the molecular chains having been attracted to each other by intermolecular forces shift in the shear direction. Once the molecular chains start to shift, some resins show a phenomenon where the molecular chains become loose and the stress decreases to a lower yield point L. After that, a region of flat stress is observed for a while, and this region is usually referred to as a necking region Rₙ. While constant stress is seen as the molecular chains are displaced by strain in the necking region Rₙ, above a predetermined strain, the molecular chains are brought closer together and closely oriented, creating a strong intermolecular force between the molecular chains, so that the stress rises steadily in the region above the predetermined strain, that is, beyond the necking region Rₙ. By extending the parison in the longitudinal axis direction until exceeding the necking region Rₙ in the first extending process, and further extending the parison, which has been extended until exceeding the necking region Rₙ, in the longitudinal axis direction with a higher internal pressure in the following second extending process, it becomes possible to manufacture a balloon catheter with the balloon for a balloon catheter in which the main orientation direction of the molecular orientation in the proximal and distal sections is the circumferential direction and a longitudinal axis direction component of the molecular orientation in the central section is more than a longitudinal axis direction component of the molecular orientation in the proximal and distal sections.

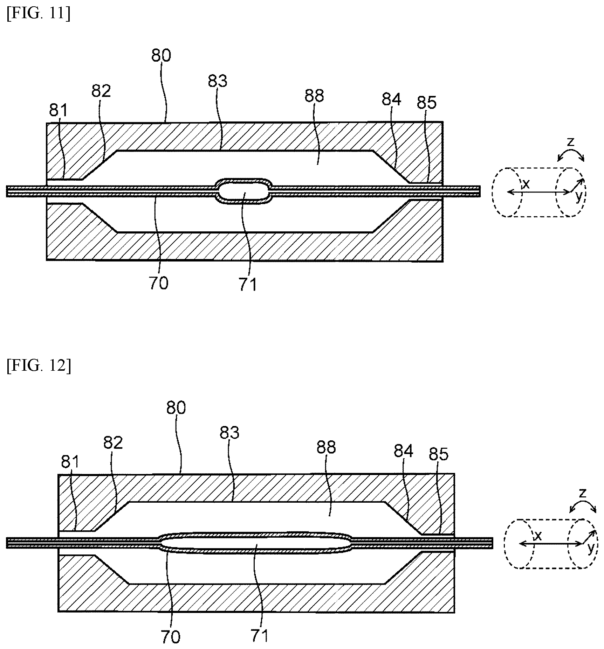

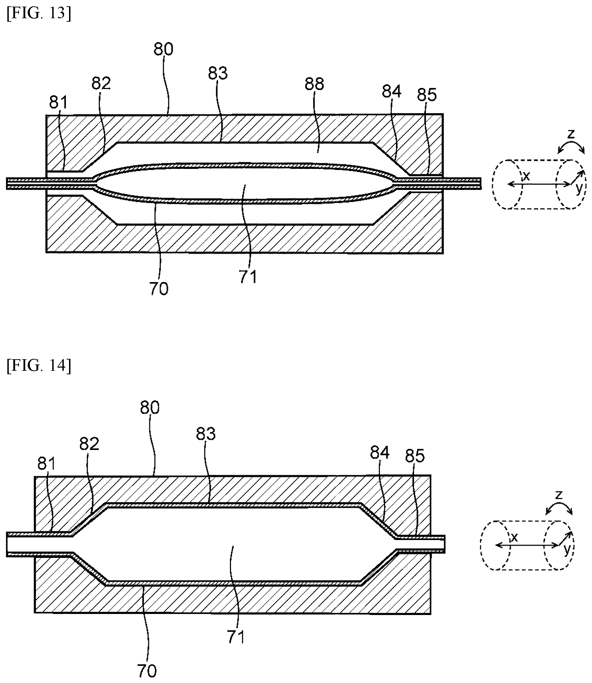

Referring to FIG. 11 to FIG. 14, the above manufacturing method will be described. FIG. 11 is a cross-sectional view showing a condition where a parison is placed in a mold in accordance with one embodiment of the present invention. FIG. 12 is a cross-sectional view showing a condition where the parison is extended in the longitudinal axis direction until exceeding the necking region of the stress-strain curve while the mold is heated in a first extending process in accordance with one embodiment of the present invention. FIG. 13 is a cross-sectional view showing a condition where the parison, which has been extended until exceeding the necking region, is further extended in the longitudinal axis direction with a higher internal pressure of the parison than in the first extending process while the mold is heated in a second extending process in accordance with one embodiment of the present invention. FIG. 14 is a cross-sectional view showing a condition after finishing the second extending process in accordance with one embodiment of the present invention.

First, a parison 70 made of a resin is prepared. The parison 70 is a tubular member having a lumen 71, and can be produced, for example, by extrusion molding. The parison 70 has one end and the other end, and extends in the longitudinal axis direction x from the one end to the other end.

Although not shown in the figures, the cross-sectional shape of the parison 70 in the direction perpendicular to the longitudinal axis direction x, that is, the radial direction y may be approximately same along the longitudinal axis direction x. Alternatively, as shown in FIG. 11, the cross-sectional shape of the parison 70 in the radial direction y may be different depending on the position in the longitudinal axis direction x. The outer diameter of a part of the parison 70, for example, the part corresponding to the straight tubular part 23, the proximal tapered part 22, and the distal tapered part 24 of the balloon 2, may be larger than the one in the other part.

As for the resin forming the parison 70, the description about the resin forming the balloon 2 in the section "1. Balloon for balloon catheter" can be referred to.

Next, a mold 80 having a lumen 88 and inner wall surface forming the lumen 88 is prepared. The inner wall surface has a straight tubular part 83, a proximal tapered part 82 located proximal to the straight tubular part 83, and a distal tapered part 84 located distal to the straight tubular part 83. The inner wall surface forming the lumen 88 of the mold 80 may have a proximal sleeve part 81 located proximal to the proximal tapered part 82 and a distal sleeve part 85 located distal to the distal tapered part 84.

The mold 80 may be formed by one member or by a plurality of members. For example, the mold 80 may be formed by a plurality of halves, or a plurality of mold members may be formed such that they are divisible in the longitudinal axis direction x.

As shown in FIG. 11, the parison 70 is placed in the lumen 88 of the mold 80. At this time, when the parison 70 has the part having a larger outer diameter, that is, when the part corresponding to the straight tubular part 23, the proximal tapered part 22, and the distal tapered part 24 of the balloon 2 has a larger outer diameter, the part is preferably positioned so that the part is located in the straight tubular part 83 of the mold 80. This makes it easy to make the part into the straight tubular part 23, the proximal tapered part 22, and the distal tapered part 24 of the balloon 2.

As shown in FIG. 12, the first extending process in which the parison 70 is extended in the longitudinal axis direction x while the mold 80 is heated is performed. At this time, the parison 70 is extended until exceeding the necking region Rₙ of a stress-strain curve of the resin forming the parison 70. As described above, the necking region Rₙ is the region showing flat stress after the yield point B and the lower yield point L, and in this region, the phenomenon in which the molecular chains of the resin that has started plastic deformation shift by stress. In the first extending process, it is important to extend the parison 70 beyond such a necking region Rₙ.

The internal pressure of the parison 70 in the first extending process is higher than the internal pressure of the parison 70 in the second extending process, and thus, in the above-described condition until exceeding the necking region Rₙ, the parison 70 can be extended in the longitudinal axis direction x while extending in the circumferential direction z is restrained.

In the case where the parison 70 is prepared in the condition where the parison 70 has been extended in the longitudinal axis direction x to some extent, such as a case where the parison 70 is prepared by extrusion molding, the amount by which the parison 70 is extended in the longitudinal axis direction x until exceeding the necking region Rₙ in the first extending process depends on the conditions of preparation of the parison 70 such as extrusion molding. Specifically, when the parison 70 has already been extended to some extent in the longitudinal axis direction x in extrusion molding or other preparation processes, the necking region Rₙ can be exceeded even if the amount by which the parison 70 is extended in the longitudinal axis direction x in the first extending process is reduced by that amount.

As shown in FIG. 13, after finishing the above first extending process, the second extending process in which the parison 70, which has been extended until exceeding the necking region Rₙ, is further extended in the longitudinal axis direction x with a higher internal pressure of the parison 70 than in the first extending process while the mold 80 is heated is performed. In the second extending process, the resin in the condition where the molecular chains of the resin have been brought closer together and closely oriented after exceeding the necking region Rₙ, is further extended in the longitudinal axis direction x. While the parison 70 is extended in the longitudinal axis direction x restraining the extension of the parison 70 in the circumferential direction z in the first extending process until exceeding the necking region Rₙ, the parison 70 is extended not only in the longitudinal axis direction x but also in the circumferential direction z in the second extending process beyond the necking region Rₙ, because the internal pressure of the parison 70 is higher in the second extending process than in the first extending process.

Thus, the balloon 2 shown in FIG. 14 can be obtained. By performing the above first extending process and second extending process, the balloon 2 can be obtained such that the main orientation direction of the molecular orientation in the proximal section 23a and the molecular orientation in the distal section 23e is the circumferential direction z, and the component in the longitudinal axis direction x of the molecular orientation in the central section 23c is more than the component in the longitudinal axis direction x of the molecular orientation in the proximal section 23a and the distal section 23e.

The stress-strain curve shown in FIG. 1 clearly shows the necking region Rₙ where the stress is constant, but depending on the resin, the region of constant stress may be short or not completely constant. In such cases, the first point where the differential coefficient of the stress-strain curve becomes 5% or more of the average change rate up to the yield point B after the stress-strain curve exceeds the yield point B is defined as the strain that exceeds the necking region Rₙ, and the first extending process can be performed until exceeding this strain.

The heating temperature in the first extending process and the second extending process can be up to around the glass transition temperature of the resin forming the balloon 2. As a heating means for the mold 80, a heater or the like generally known can be appropriately used.

In the first extending process, the inside of the parison 70 is preferably pressurized by introducing fluid into the lumen 71 of the parison 70, and the pressure is preferably 3 MPa or less. Alternatively, the lumen 71 of the parison 70 and the outside of the parison 70 may have the same pressure, and in other words, the lumen 71 of the parison 70 may be unpressurized.

In the second extending process, the inside of the parison 70 is preferably pressurized by introducing fluid into the lumen 71 of the parison 70, and the pressure is higher than the internal pressure of the parison 70 in the first extending process, and for example, the pressure is preferably 1 MPa or more, more preferably 1.5 MPa or more, and even more preferably 2 MPa or more, and preferably 5 MPa or less, more preferably 4.5 MPa or less, even more preferably 4 MPa or less, and may be 3 MPa or less.

Preferably, the parison 70 is unpressurized in the first extending process, and pressurization of the parison 70 starts after exceeding the necking region Rₙ in the second extending process. This makes it easier to obtain the balloon 2 in which the main orientation direction of the molecular orientation in the proximal section 23a and the molecular orientation in the distal section 23e is the circumferential direction z, and the component in the longitudinal axis direction x of the molecular orientation in the central section 23c is more than the component in the longitudinal axis direction x of the molecular orientation in the proximal section 23a and the distal section 23e.

The mold 80 is preferably heated so that the center portion of the straight tubular part 83 of the mold 80 is the hottest. This makes it easier to obtain the balloon 2 in which the component in the longitudinal axis direction x of the molecular orientation in the central section 23c is more than the component in the longitudinal axis direction x of the molecular orientation in the proximal section 23a and the distal section 23e, and also makes it easier to make the film thickness of the balloon 2 in the central section 23c thinner than the film thickness of the balloon 2 in the proximal section 23a and the distal section 23e.

The present application claims priority based on Japanese Patent Application No. 2021-79672 filed on May 10, 2021.

### EXAMPLES

Hereinafter, the present invention will be described with reference to Examples. The present invention is not limited by the following Examples, can be absolutely carried out with appropriate changes to the Examples within a scope in compliance with the intent described above and later, and all the changes are to be encompassed within the technical scope of the present invention.

### Example 1

A parison was produced by extrusion molding polyamide 12. The parison was placed in a mold, and the parison was extended in the longitudinal axis direction until exceeding the necking region of the stress-strain curve with an inner pressure of 2 MPa to the parison while the mold was heated at 70°C. Next, the parison was extended in the longitudinal axis direction with an inner pressure of 4.3 MPa to the parison while the mold was heated at 70°C, and a balloon was obtained.

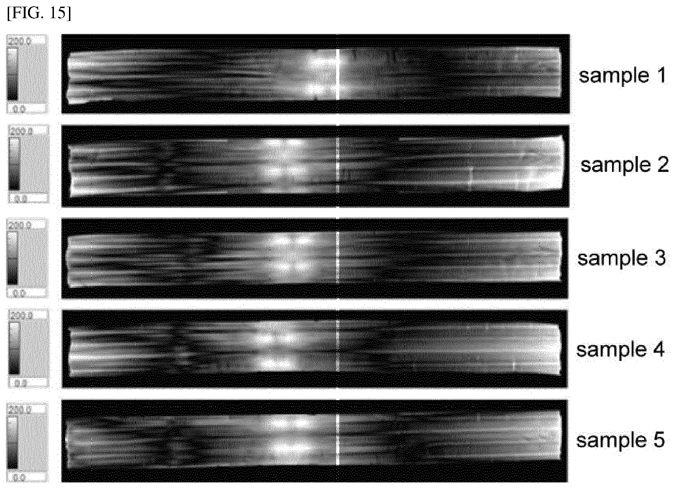

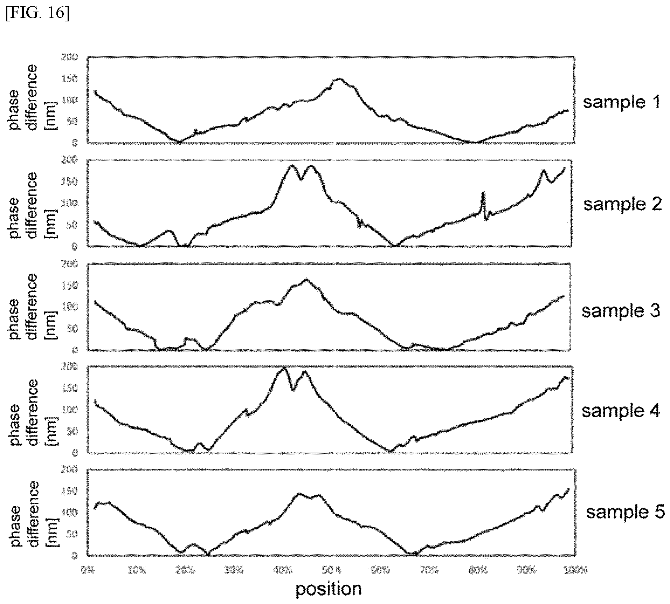

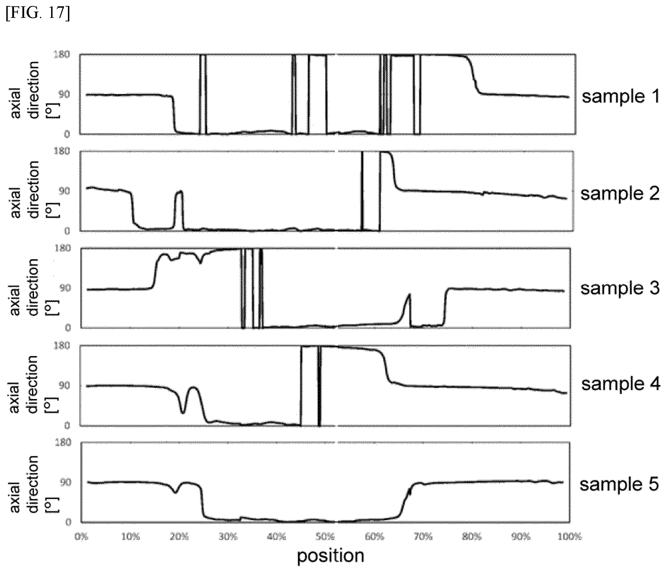

By the same method, five balloons were manufactured. For each balloon, the proximal tapered part and the distal tapered part were cut off to obtain a tubular-shaped straight tubular part as shown in FIG. 7, and cut open the tubular-shaped straight tubular part along the cut line in the longitudinal axis direction to obtain rectangular samples 1 to 5. The samples 1 to 5 were measured with a two-dimensional birefringence evaluation system WPA-100 manufactured by Photonic Lattice, Inc. to obtain the molecular orientation. Results are shown in FIG. 15 to FIG. 17. In each of FIG. 15 to FIG. 17, the data of the samples 1 to 5 are shown from top to bottom. In each balloon, the main orientation direction of the molecular orientation in the central section of the straight tubular part was the longitudinal axis direction, and the balloons in which the main orientation direction of the molecular orientation in the proximal section and distal section of the straight tubular part was the circumferential direction were stably obtained.

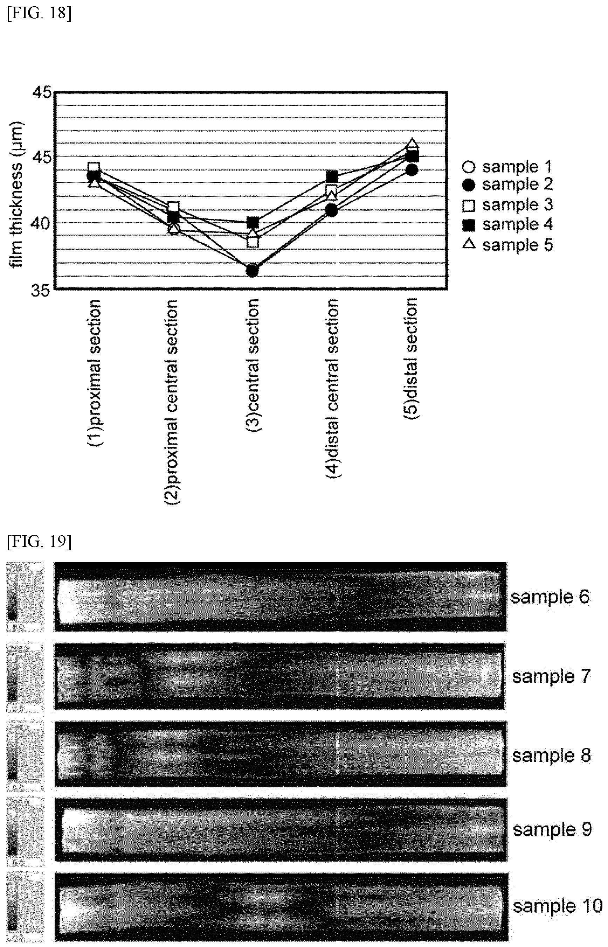

For the above five balloons, the film thicknesses of the proximal section (1), the proximal central section (2), the central section (3), the distal central section (4), and the distal section (5) were measured using a spline micrometer SPM2-25MX manufactured by Mitutoyo Corporation. Results are shown in FIG. 18. Each balloon had the thinnest film thickness in the central section, and the balloons in which the film thickness became thicker from the central section to the proximal and distal sides were stably obtained.

In the same manner as in Example 1, another 30 balloons were manufactured. Internal pressure was continuously applied to these 30 balloons until they were fractured, and the condition of fracture was observed after they were fractured to determine whether circumferential fracture occurred or not. No circumferential fracture occurred in any of the balloons.

### Comparative example 1

A parison was prepared in the same manner as in Example 1. The parison was placed in the same mold as Example 1, and the parison was extended in the longitudinal axis direction with an inner pressure of 3.5 MPa to the parison while the mold was heated at 60°C to obtain a balloon.

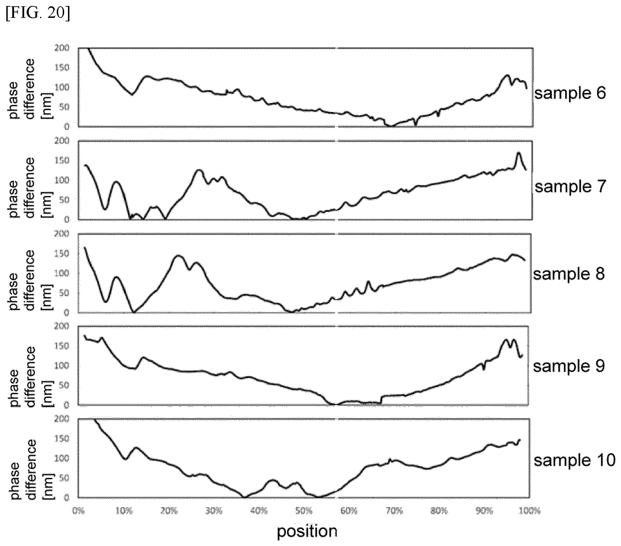

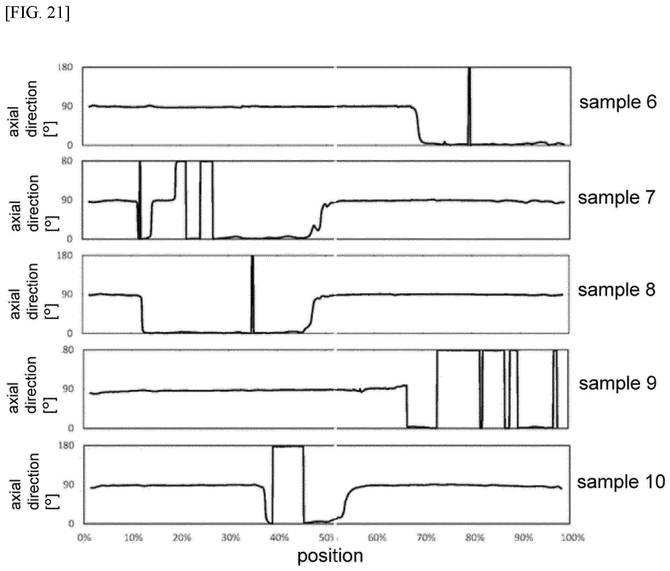

By the same method, five balloons were manufactured. For each balloon, the proximal tapered part and the distal tapered part were cut off to obtain a tubular-shaped straight tubular part as shown in FIG. 7, and cut open the tubular-shaped straight tubular part along the cut line in the longitudinal axis direction to obtain rectangular samples 6 to 10. The samples 6 to 10 were measured with a two-dimensional birefringence evaluation system WPA-100 manufactured by Photonic Lattice, Inc. to obtain the molecular orientation. Results are shown in FIG. 19 to FIG. 21. In each of FIG. 19 to FIG. 21, the data of the samples 6 to 10 are shown from top to bottom. There was variation in orientation from balloon to balloon, and in all balloons, the longitudinal axis direction component of the molecular orientation in the central section of the straight tubular part was not more than the longitudinal axis direction component of the molecular orientation in the proximal section and the distal section.

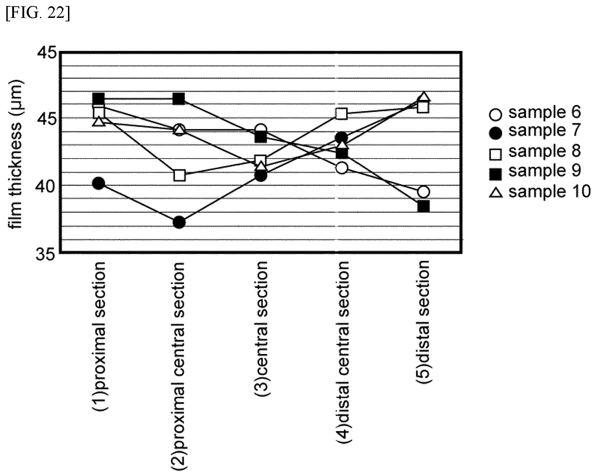

For the above five balloons, the film thicknesses of the proximal section (1), the proximal central section (2), the central section (3), the distal central section (4), and the distal section (5) were measured using a spline micrometer SPM2-25MX manufactured by Mitutoyo Corporation. Results are shown in FIG. 22. The tendency of film thickness in the longitudinal axis direction varied from balloon to balloon, which showed that the method in Comparative example 1 could not control the film thickness in the longitudinal axis direction.

In the same manner as in Comparative example 1, another 30 balloons were manufactured. Internal pressure was continuously applied to these 30 balloons until they were fractured, and the condition of fracture was observed after they were fractured to determine whether circumferential fracture occurred or not. Three of the 30 balloons had circumferential fracture.

### DESCRIPTION OF REFERENCE SIGNS

1: balloon catheter
2: balloon
3: shaft
4: hub
5: guidewire insertion port
6: fluid inlet
21: proximal sleeve part
22: proximal tapered part
23: straight tubular part
23a: proximal section
23b: proximal central section
23c: central section
23d: distal central section
23e: distal section
24: distal tapered part
25: distal sleeve part
70: parison
71: lumen of parison
80: mold
81: proximal sleeve part of mold
82: proximal tapered part of mold
83: straight tubular part of mold
84: distal tapered part of mold
85: distal sleeve part of mold
88: lumen of mold
B: yield point
D₀: position of 0%
D₁₀: position of 10%
D₄₀: position of 40%
D₆₀: position of 60%
D₉₀: position of 90%
D₁₀₀: position of 100%
L: lower yield point
Rₙ: necking region
S₁: first cut line
S₂: second cut line
x: longitudinal axis direction
y: radial direction
z: circumferential direction

## Claims

1. A balloon (2) for a balloon catheter (1), the balloon (2) made of a resin having molecular orientation, having a longitudinal axis direction and a circumferential direction that is along a circumference of the balloon (2) in an inflated state in a cross section perpendicular to the longitudinal axis direction, comprising:
a straight tubular part (23),
a proximal tapered part (22) located proximal to the straight tubular part (23), and
a distal tapered part (24) located distal to the straight tubular part (23), wherein
a proximal end of the straight tubular part (23) is defined as a position of 0%, and a distal end of the straight tubular part (23) is defined as a position of 100% in the longitudinal axis direction;
a main orientation direction of the molecular orientation in a proximal section (23a) from the position of 0% to a position of 10% and a distal section (23e) from a position of 90% to the position of 100% is the circumferential direction; and
a longitudinal axis direction component of the molecular orientation in a central section (23c) from a position of 40% to a position of 60% is more than a longitudinal axis direction component of the molecular orientation in the proximal section (23a) and the distal section (23e).

2. The balloon (2) for a balloon catheter (1) according to claim 1, wherein a main orientation direction of the molecular orientation in the central section is the longitudinal axis direction.

3. The balloon (2) for a balloon catheter (1) according to claim 1 or 2, wherein the longitudinal axis direction component of the molecular orientation gradually decreases from the central section (23c) toward the position of 0% and gradually decreases from the central section (23c) toward the position of 100%.

4. The balloon for a balloon catheter (1) according to claim 2 or 3, wherein
a section from the position of 10% to a position of 40% is defined as a proximal central section (23b) and a section from a position of 60% to the position of 90% is defined as a distal central section (23d); and
a main orientation direction of the molecular orientation in the proximal central section (23b) and the distal central section (23d) changes from the longitudinal axis direction to the circumferential direction.

5. The balloon (2) for a balloon catheter (1) according to any one of claims 1 to 4,
wherein a film thickness of the balloon (2) in the central section (23c) is thinner than a film thickness of the balloon (2) in the proximal section (23a) and the distal sectior (23e).

6. A balloon catheter (1), comprising the balloon (2) according to any one of claims 1 to 5.

7. A method of manufacturing the catheter according to claim 6, comprising
a step of preparing a parison (70) made of a resin;
a step of preparing a mold (80) having a lumen (88) and an inner wall surface forming the lumen (88), the inner wall surface having a straight tubular part (83), a proximal tapered part (82) located proximal to the straight tubular part (83), and a distal tapered part (84) located distal to the straight tubular part (83),
a step of placing the parison (70) in the mold (80);
a step of a first extending process in which the parison (70) is extended in the longitudinal axis direction until exceeding a necking region of a stress-strain curve while the mold (80) is heated; and
a step of a second extending process in which, after the first extending process, the parison (70), which has been extended until exceeding the necking region, is further extended in the longitudinal axis direction with a higher internal pressure of the parison (70) than in the first extending process while the mold (80) is heated.

8. The method of manufacturing the balloon catheter (1) according to claim 7,
wherein the parison (70) is pressurized at a lower pressure in the first extending process than in the second extending process, and is further pressurized after exceeding the necking region in the second extending process.

9. The method of manufacturing the balloon catheter (1) according to claim 7 or 8, comprising a step of heating the mold (80) so that a center portion of the straight tubular part (83) of the mold (80) is the hottest.

## Patentansprüche

1. Ballon (2) für einen Ballonkatheter (1), wobei der Ballon (2), der aus einem Harz, das eine molekulare Orientierung hat, hergestellt ist, eine Längsachsenrichtung und eine Umfangsrichtung hat, die in einem aufgeblähten Zustand entlang eines Umfangs des Ballons (2) in einem Querschnitt senkrecht zur Längsachsenrichtung verläuft, aufweisend:
einen geradlinigen röhrenförmigen Teil (23),
einen sich verjüngenden proximalen Teil (22), der proximal bezüglich des geradlinigen röhrenförmigen Teils (23) angeordnet ist, und
einen sich verjüngenden distalen Teil (24), der distal bezüglich des geradlinigen röhrenförmigen Teils (23) angeordnet ist,
wobei
in der Längsachsenrichtung ein proximales Ende des geradlinigen röhrenförmigen Teils (23) als eine Position 0% definiert ist und ein distales Ende des geradlinigen röhrenförmigen Teils (23) als eine Position 100% definiert ist,
eine Hauptorientierungsrichtung der molekularen Orientierung in einem proximalen Abschnitt (23a) von der Position 0% zu einer Position 10% und in einem distalen Abschnitt (23e) von einer Position 90% zu der Position 100% die Umfangsrichtung ist; und
eine Längsachsenrichtungskomponente der molekularen Orientierung in einem zentralen Abschnitt (23c) von einer Position 40% zu einer Position 60% größer ist als eine Längsachsenrichtungskomponente der molekularen Orientierung in dem proximalen Abschnitt (23a) und dem distalen Abschnitt (23e).

2. Ballon (2) für einen Ballonkatheter (1) nach Anspruch 1, wobei eine Hauptorientierungsrichtung der molekularen Orientierung in dem zentralen Abschnitt die Längsachsenrichtung ist.

3. Ballon (2) für einen Ballonkatheter (1) nach Anspruch 1 oder 2, wobei die Längsachsenrichtungskomponente der molekularen Orientierung von dem zentralen Abschnitt (23c) hin zu der Position 0% allmählich kleiner wird und von dem zentralen Abschnitt (23c) hin zu der Position 100% allmählich kleiner wird.

4. Ballon (2) für einen Ballonkatheter (1) nach Anspruch 2 oder 3, wobei
ein Abschnitt von der Position 10% zu einer Position 40% als ein proximaler zentraler Abschnitt (23b) definiert ist, und
ein Abschnitt von einer Position 60% zu der Position 90% als ein distaler zentraler Abschnitt (23d) definiert ist; und
eine Hauptorientierungsrichtung der molekularen Orientierung in dem proximalen zentralen Abschnitt (23b) und in dem distalen zentralen Abschnitt (23d) sich von der Längsachsenrichtung in die Umfangsrichtung ändert.

5. Ballon (2) für einen Ballonkatheter (1) nach einem der Ansprüche 1 bis 4, wobei eine Foliendicke des Ballons (2) in dem zentralen Abschnitt (23c) dünner ist als eine Foliendicke des Ballons (2) in dem proximalen Abschnitt (23a) und dem distalen Abschnitt (23e).

6. Ballonkatheter (1) aufweisend den Ballon (2) nach einem der Ansprüche 1 bis 5.

7. Verfahren zur Herstellung des Ballonkatheters nach Anspruch 6, aufweisend:
einen Schritt zum Zurichten eines aus einem Harz hergestellten Vorformlings (70);
einen Schritt zum Zurichten eines Formwerkzeugs (80), das ein Lumen (88) und eine das Lumen (88) bildende Innenwandfläche hat, wobei die Innenwandfläche aufweist: einen geradlinigen röhrenförmigen Teil (83), einen sich verjüngenden proximalen Teil (82), der proximal bezüglich des geradlinigen röhrenförmigen Teils (83) angeordnet ist, und einen sich verjüngenden distalen Teil (84), der distal bezüglich des geradlinigen röhrenförmigen Teils (83) angeordnet ist,
einen Schritt zum Anordnen des Vorformlings (70) in dem Formwerkzeug (80);
einen Schritt eines ersten Streckprozesses, in dem der Vorformling (70) in der Längsachsenrichtung bis Überschreiten eines Einschnürungsbereichs einer Spannungs-Dehnungs-Kennlinie gestreckt wird, während das Formwerkzeug (80) erhitzt wird; und
einen Schritt eines zweiten Streckprozesses, in dem nach dem ersten Streckprozess der Vorformling (70), der bis Überschreiten des Einschnürungsbereichs gestreckt worden ist, in der Längsachsenrichtung mit einem höheren Innendruck des Vorformlings (70) als in dem ersten Streckprozess weiter gestreckt wird, während das Formwerkzeug (80) erhitzt wird.

8. Verfahren zur Herstellung des Ballonkatheters (1) nach Anspruch 7, wobei der Vorformling (70) in dem ersten Streckprozess unter einen niedrigeren Druck gesetzt wird als in dem zweiten Streckprozess und nach Überschreiten des Einschnürungsbereichs in dem zweiten Streckprozess weiter unter Druck gesetzt wird.

9. Verfahren zur Herstellung des Ballonkatheters (1) nach Anspruch 7 oder 8, aufweisend einen Schritt zum Erhitzen des Formwerkzeugs (80), derart, dass eine zentrale Zone des geradlinigen röhrenförmigen Teils (83) des Formwerkzeugs (80) die heißeste ist.

## Revendications

1. Ballonnet (2) pour un cathéter à ballonnet (1), le ballonnet (2) étant constitué d'une résine ayant une orientation moléculaire, ayant une direction d'axe longitudinal et une direction circonférentielle qui est le long d'une circonférence du ballonnet (2) à l'état gonflé dans une section transversale perpendiculaire à la direction d'axe longitudinal, comprenant :
une partie tubulaire droite (23), une partie conique proximale (22) située proximalement à la partie tubulaire droite (23), et une partie conique distale (24) située distalement à la partie tubulaire droite (23), dans lequel une extrémité proximale de la partie tubulaire droite (23) est définie comme une position de 0 %, et une extrémité distale de la partie tubulaire droite (23) est définie comme une position de 100 % dans la direction d'axe longitudinal ;
une direction d'orientation principale de l'orientation moléculaire dans une section proximale (23a) de la position de 0 % à une position de 10 % et une section distale (23e) d'une position de 90 % à la position de 100 % est la direction circonférentielle ; et
une composante de direction d'axe longitudinal de l'orientation moléculaire dans une section centrale (23c) d'une position de 40 % à une position de 60 % est plus qu'une composante de direction d'axe longitudinal de l'orientation moléculaire dans la section proximale (23a) et la section distale (23e).

2. Ballonnet (2) pour un cathéter à ballonnet (1) selon la revendication 1, dans lequel une direction d'orientation principale de l'orientation moléculaire dans la section centrale est la direction d'axe longitudinal.

3. Ballonnet (2) pour un cathéter à ballonnet (1) selon la revendication 1 ou 2, dans lequel la composante de direction d'axe longitudinal de l'orientation moléculaire diminue progressivement à partir de la section centrale (23c) vers la position de 0 % et diminue progressivement à partir de la section centrale (23c) vers la position de 100 %.

4. Ballonnet pour un cathéter à ballonnet (1) selon la revendication 2 ou 3, dans lequel
une section de la position de 10 % à une position de 40 % est définie comme une section centrale proximale (23b) et une section d'une position de 60 % à la position de 90 % est définie comme une section centrale distale (23d) ; et
une direction d'orientation principale de l'orientation moléculaire dans la section centrale proximale (23b) et la section centrale distale (23d) change de la direction d'axe longitudinal à la direction circonférentielle.

5. Ballonnet (2) pour un cathéter à ballonnet (1) selon l'une quelconque des revendications 1 à 4, dans lequel une épaisseur de film du ballonnet (2) dans la section centrale (23c) est plus fine qu'une épaisseur de film du ballonnet (2) dans la section proximale (23a) et la section distale (23e)

6. Cathéter à ballonnet (1), comprenant le ballonnet (2) selon l'une quelconque des revendications 1 à 5.

7. Procédé de fabrication du cathéter selon la revendication 6, comprenant une étape de préparation d'une paraison (70) en résine ; une étape de préparation d'un moule (80) ayant une lumière (88) et une surface de paroi intérieure formant la lumière (88), la surface de paroi intérieure ayant une partie tubulaire droite (83), une partie conique proximale (82) située proximalement à la partie tubulaire droite (83), et une partie conique distale (84) située distalement à la partie tubulaire droite (83), une étape de mise en place de la paraison (70) dans le moule (80) ; une étape d'un premier processus d'extension dans lequel la paraison (70) est étendue dans la direction d'axe longitudinal jusqu'à dépasser une région de rétrécissement d'une courbe contrainte-déformation tandis que le moule (80) est chauffé ; et
une étape d'un deuxième processus d'extension dans lequel, après le premier processus d'extension, la paraison (70), qui a été étendue jusqu'à dépasser la région de rétrécissement, est étendue davantage dans la direction d'axe longitudinal avec une pression interne de la paraison (70) plus élevée que dans le premier processus d'extension pendant que le moule (80) est chauffé.

8. Procédé de fabrication du cathéter à ballonnet (1) selon la revendication 7, dans lequel la paraison (70) est pressurisée à une pression moins élevée dans qle premier processus d'extension que dans le deuxième processus d'extension, et est en outre pressurisée après avoir dépassé la région de rétrécissement dans le deuxième processus d'extension.

9. Procédé de fabrication du cathéter à ballonnet (1) selon la revendication 7 ou 8, comprenant une étape de chauffage du moule (80) de sorte qu'une portion centrale de la partie tubulaire droite (83) du moule (80) soit la plus chaude.
